# EUROPEAN PATENT SPECIFICATION

(11) **EP 3 673 803 B1**
(45) Date of publication and mention of the grant of the patent: **08.12.2021**
(21) Application number: 19000567.8
(22) Date of filing: 18.12.2019
(51) Int. Cl.: A61B 5/11, A61B 5/16, A61B 5/00, A63F 9/10, G06F 3/14

(54) **APPARATUS FOR MEASURING HUMAN PSYCHOPHYSICAL PERFORMANCE**
VORRICHTUNG ZUR MESSUNG MENSCHLICHER PSYCHOPHYSISCHER LEISTUNG
APPAREIL POUR MESURER LA PERFORMANCE PSYCHOPHYSIQUE HUMAINE

(30) Priority: 27.12.2018 PL 42837218
(43) Date of publication of application: 01.07.2020
(73) Proprietor: Politechnika Slaska, 44-100 Gliwice (PL)
(72) Inventor: Dobrowolska, Malgorzata, 40-540 Katowice (PL); Gzik, Marek, 44-100 Gliwice (PL); Wodarski, Piotr, 43-190 Mikolow (PL); Jedrasiak, Karol, 44-190 Mikolow (PL); Nawrat, Aleksander, 41-807 Zabrze (PL); Szabo, Stanislav, 044 41 Sady Nad Torysou (SK); Kalavsky, Peter, 04431 Druzstevna PRI H (SK)

(56) References cited:
- EP-A2- 2 033 695
- CN-A- 108 564 829
- CN-U- 207 384 817
- KR-B1- 101 271 421
- US-A1- 2006 265 974

## Description

The subject of the invention is an apparatus for measuring psycho- physical ability of an individual.

The present solution may be used in all psychological testing created in academic, experimental versions available on the market as well as for individual use by a participant in assessment, psychological offices where their own tests are being developed and they create an original process of measuring psychological variables. The system consists elements of a puzzle provided with press buttons, connected with each other, a control device allowing connection with, for example, a computer or recording responses on memory storage media and a tracking system for upper limbs movements and recording of facial expressions when test tasks are being performed.

The number of the puzzle elements depends on the score scale of a given test and is within the range from 2 to 10 elements with press buttons. Pressing on relevant button on each of the puzzle allows, due to the connection with the computer via wired or wireless interfaces, the measurement of the tested variables in different scales, from a 2-degree to 10-degree scale.

The system may be used in testing with the use of different types of scales, with different configuration of the puzzle elements with buttons. Depending on the scale chosen, the configuration of the elements with the buttons is selected for the participant, the pressing thereon will result in indicating the relevant test response and calculation of relevant score. That option is possible for individual and group testing. Any configuration of the scale, i.e. from 2 to 10 allows performance of each psychological assessment, independently from a type of scale and responses.. Moreover, the system allows display on the mobile buttons of the puzzle and arranging thereof by moving the elements. The correctness of the configuration is examined due to the use of the RFID system (Radio-frequency identification).

Additionally, due to the use of cameras the system allows registration of the trajectory of the upper limbs movements, which allows assessment of human reactions to the question asked. Due to the camera in the rear part, it is also possible to automatically assess facial expressions during the test performance. The entire system is a portable, mobile platform the advantage thereof is the possibility to perform assessment both in the psychologist's office and at the client's premises.

A solution similar to the present apparatus is the system "Puzzle comprising timer" no. GB 2499584, in the system thereof tasks are solved related to arranging spatial puzzles with simultaneous detection of the time spent on arranging particular figures. Such a solution is limited to only measurement of the time for solving the task at a defined level of difficulty. Somewhat different approach towards performance of similar tests is known from the patent "Computer input device enabling three degrees of freedom and related input and feedback methods" no. US 2007/014.6325 A1 or "Audiotactile vision substitution system" no. US 2008/0058894 A1 where only a computer system has been used to perform the tests.

Another solutions are EP2033695A2 Radio frequency (RF) sensing system, RF display device, and puzzle system using the same and KR101271421B1 Contents providing apparatus using puzzle recognition. Both solutiones disclose system using puzzle recognition.

Another example state of the art is CN 207384817.

It is known US 2006/265974, which is a toy for children for completely different use.

It is also known CN 108564829 which relates to the field of medical devices, and more particularly to an auxiliary treatment device for autistic patients.

There is no apparatus to measure psycho-physical ability of an individual in a form of a reconfigured puzzle with interactive displays and a system to verify the correctness of the performed tasks. Also, there are no solutions based on reconfigured controllers to give questionnaire response. Universality of the present apparatus combines those two functionalities into one system. The majority of prior art solutions is based only on computer technology. The present solution allows performance of plurality of tests during one assessment session, allows introduction of difficulties both in a visual and mechanical form. The solution contributes to the reduction of time of testing in comparison with the currently used assessment methods. The apparatus combines the advantages and functionalities of plurality of research methods being used into one system, which reduces the costs to be incurred in the case of contraction of a psychological research and testing office. The invention is defined by an apparatus according to claim 1. Embodiments are defined by dependent claims **2-11**.

The subject of the invention is an apparatus for measuring human psychophysical performance designed on the basis of a board with sensors where reconfigured elements are arranged in a form of a puzzle. The puzzle includes RFID (radio-frequency identification) elements that are identified in a matrix of aerials built into the board. The system verifies the correctness of the tasks performed by detection of the arrangement of each of the elements. Moreover, the puzzle is provided with the press button which allows selection of the response or modification of the task configuration. Due to this functionality the apparatus may be used as a reconfigured controller for giving questionnaire responses. On the board there are mounted cameras tracking facial expressions of the participant and the movements of the upper limbs thereof, which allows assessment of the coordination thereof.

The application of the present solution includes tests performance in a form of arranging images, patterns and other graphic elements, also the apparatus may be used as a reconfigured controller with press buttons where responses to a given task may be displayed and a choice of a response may be made by pressing the button located on each puzzle elements.

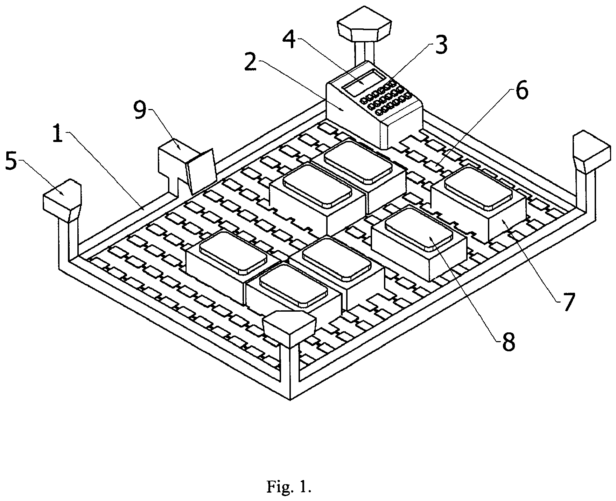

The subject of the invention is presented in the preferred embodiment in Figure 1 which shows an axonometric view of the position.

The present solution according to Figure 1 comprises a board 1 where in one of the corners is located a controller 2 with press buttons 3 and a display 4. On the display 4 the content of the task to be performed is displayed. In the corners of the board 1 on extensions there are cameras 5 to analyse the movements of the participant. In the base of the board 1 built are aerial sensors 6 working in the RFID system. The aerial sensors 6 are arranged in a matrix row. The sensors 6 are away at least 2 cm from each other. On the board 1 there is arranged a puzzle elements 7 consisting of a display with a press button 8. Aerial sensors 6 consist of a built-in RFID system, on the board 1 there is arranged the puzzle elements.

The puzzle elements 7 consists a built-in system of RFID receivers due to which the aerial sensors 6 localise the position thereof. On the display with the press button 8 presented is a part of the puzzle or displayed is a photography with the response that is activated by pressing the button 8 with the display . A rear wall of the board 1 consists of a camera 9 configured to track facial expressions.

The assessment with the use of the present apparatus is by moving the puzzle 7 on the panel 1 above the aerial sensors 6. On the display with the button 8 displayed is a part of the puzzle. The content of the task is presented on the panel 4 of the controller 2. The aerial sensors 6 localise the position of the puzzle elements 7. The cameras 5 read out the trajectory of the movements of the participant's limbs. The camera 9 reads out the facial expressions. The controller 2 collects in a wired way the data from the camera 5 and 9 and from the aerial sensors 6. In the wired way the data from the controller 2 is transmitted to the computer. In the case of using the apparatus as a panel with buttons for testing, on the display 4 appears a content of the question and the puzzle elements 7 with the button 8 shows a content of the answer. The selection of the answer is by pressing on the display with the button 8. The results are memorised in the memory of the controller 2. The speed of giving the response is registered by the controller. Kinematics of the movements of the limbs during selection of the answer is registered by the camera 5 and the facial expressions by the camera 9.

## Claims

1. An apparatus for measuring psycho-physical ability of an individual, designed in a form of a board (1) having corners, a controller (2) and moveable puzzle elements, each puzzle element (7) consisting of a display and a press button, the apparatus comprises the board (1), where in one of the corners is located the controller (2) with press buttons (3) and a display (4), the corners of the board having extensions, the extensions having cameras(5) mounted thereon configured to analyse the movements of the participant, built in a base of the board (1) are aerial sensors (6) comprising an RFID system, on the board there is arranged the puzzle elements (7) with the display configured to present a part of a puzzle or display a photography with a response that is activated by pressing the button with the display, a rear wall of the board (1) consisting of a camera (9) configured to track facial expressions, wherein each puzzle element (7) contains a built-in system of RFID receivers, the aerial sensors (6) being configured to localize the position of the RFID receivers, the press button (8) being configured to receive user input by pressing, the controller further containing a memory configured to save the results of the received user input.

2. The apparatus according to claim 1, **characterised in that** the puzzle elements (7) are moveable on the board (1) above the aerial sensors (6).

3. The apparatus according to claim 1, **characterised in that** the puzzle elements (7) consists of the receiver of the RFID system which sends the ID identification wirelessly to the aerial sensors (6).

4. The apparatus according to claim 1, **characterised in that** aerial sensors (6) located in the board (1) are arranged in a matrix way and away at least 2 cm from each other.

5. The apparatus according to claim 1, **characterised in that** in each of the corners of the board (1) located on an extension is the cameras (5) constituting a system to analyse the movements of the upper limbs.

6. The apparatus according to claim 1, **characterised in that** the extensions the cameras (5) are located thereon are from 5 cm to 50 cm long.

7. The apparatus according to claim 1, **characterised in that** the width and length of the sides of the board (1) are within the range from 50 cm to 150 cm.

8. The apparatus according to claim 1, **characterised in that** the puzzle element (7) is at least 2 cm high.

9. The apparatus according to claim 1, **characterised in that** the communication of the puzzle elements (7) with the controller (2) is performed in a wireless way.

10. The apparatus according to claim 1, **characterised in that** the communication of the cameras (5) with the controller (2) is performed in a wired way.

11. The apparatus according to claim 1, **characterised in that** the communication of the camera (9) with the controller (2) is performed in a wired way.

## Patentansprüche

1. Vorrichtung zur Messung der psychophysischen Fähigkeiten einer Person, die in Form eines Brettes (1) mit Ecken, eine Steuergerät (2) und beweglichen Rätselelementen gestaltet ist, wobei jedes Rätselelement (7) aus einer Anzeige und einem Druckknopf besteht, die Vorrichtung das Brett umfasst (1), in dem sich in einer der Ecken der Steuergerät (2) mit Druckknöpfen (3) und einer Anzeige (4) befindet, wobei die Ecken des Brettes Verlängerungen aufweisen, wobei an den Verlängerungen Kameras (5) angebracht sind, die so konfiguriert sind, dass sie die Bewegungen des Probanden analysieren, wobei in einer Basis des Brettes (1) Antennensensoren (6) eingebaut sind, die ein RFID-System umfassen, auf dem Brett die Rätselelemente (7) mit dem Display angeordnet sind, das so konfiguriert ist, dass es einen Teil eines Rätsels präsentiert oder eine Fotografie mit einer Antwort anzeigt, die durch Drücken des Druckknopfes mit dem Display aktiviert wird, wobei eine Rückwand des Brettes (1) aus einer Kamera (9) besteht, die so konfiguriert ist, dass sie Gesichtsausdrücke verfolgt, wobei jedes Rätselelement (7) ein eingebautes System von RFID-Empfängern enthält, wobei die Antennensensoren (6) so konfiguriert sind, dass sie die Position der RFID-Empfänger lokalisieren, wobei der Druckknopf (8) so konfiguriert ist, dass er durch Drücken eine Benutzereingabe empfängt, wobei der Steuergerät ferner einen Speicher enthält, der so konfiguriert ist, dass er die Ergebnisse der empfangenen Benutzereingabe speichert.

2. Vorrichtung nach Anspruch 1, **dadurch gekennzeichnet, dass** die Rätselelemente (7) auf dem Brett (1) oberhalb der Antennensensoren (6) beweglich sind.

3. Vorrichtung nach Anspruch 1, **dadurch gekennzeichnet, dass** die Rätselelemente (7) aus dem Empfänger des RFID-Systems bestehen, der die ID-Kennung drahtlos an die Antennensensoren (6) sendet.

4. Vorrichtung nach Anspruch 1, **dadurch gekennzeichnet, dass** die in dem Brett (1) befindlichen Antennensensoren (6) matrixförmig und mit einem Abstand von mindestens 2 cm zueinander angeordnet sind.

5. Vorrichtung nach Anspruch 1, **dadurch gekennzeichnet, dass** sich in jeder der Ecken des Brettes (1) auf einer Verlängerung die Kameras (5) befinden, die ein System zur Analyse der Bewegungen der oberen Gliedmaßen bilden.

6. Vorrichtung nach Anspruch 1, **dadurch gekennzeichnet, dass** die Verlängerungen, an denen die Kameras (5) angebracht sind, zwischen 5 cm und 50 cm lang sind.

7. Vorrichtung nach Anspruch 1, **dadurch gekennzeichnet, dass** die Breite und Länge der Seiten des Brettes (1) im Bereich von 50 cm bis 150 cm liegen.

8. Vorrichtung nach Anspruch 1, **dadurch gekennzeichnet, dass** das Rätselelement (7) mindestens 2 cm hoch ist.

9. Vorrichtung nach Anspruch 1, **dadurch gekennzeichnet, dass** die Kommunikation der Rätselelemente (7) mit dem Steuergerät (2) drahtlos erfolgt.

10. Vorrichtung nach Anspruch 1, **dadurch gekennzeichnet, dass** die Kommunikation der Kameras (5) mit dem Steuergerät (2) drahtgebunden erfolgt.

11. Vorrichtung nach Anspruch 1, **dadurch gekennzeichnet, dass** die Kommunikation der Kamera (9) mit dem Steuergerät (2) drahtgebunden erfolgt.

## Revendications

1. Dispositif de mesure des capacités psychophysiques humaines, conçu sous forme de plateau (1) possédant des coins, un contrôleur (2) et des éléments mobiles du casse-tête, chaque élément dudit casse-tête (7) se composant d'un écran et d'un bouton, ledit dispositif est composé d'un plateau (1) sur lequel, dans un de ses coins, est installé un contrôleur (2) avec des boutons (3) et avec un écran (4), lesdits coins du plateau sont munis de rallonges, sur lesdites rallonges sont installées les caméras (5) configurées pour analyser les mouvements d'une personne examinée, dans le socle dudit plateau (1) sont intégrés les capteurs avec antenne (6) et avec le système RFID intégré, sur ledit plateau sont placés les éléments du casse-tête (7) avec l'écran configuré pour présenter des fragments du casse-tête ou afficher une photographie avec une réponse qui est activée par une pression dudit bouton avec écran, sur la face arrière dudit plateau (1) se trouve la caméra (9) configurée pour suivre la mimique du visage, chacun élément dudit casse-tête (7) comportant le système des récepteurs RFID intégré, lesdits capteurs avec antenne (6) sont configurés pour localiser la position des récepteurs RFID, ledit bouton (8) est configuré pour recevoir une entrée fournie par l'utilisateur par sa pression et ledit contrôleur comprend une mémoire configurée pour enregistrer les résultats de l'entrée fournie par l'utilisateur.

2. Dispositif selon la revendication 1, **caractérisé en ce que** lesdits éléments du casse-tête (7) peuvent être déplacés sur le plateau (1) au-dessus des capteurs avec antenne (6).

3. Dispositif selon la revendication 1, **caractérisé en ce que** lesdits éléments du casse-tête (7) se composent d'un récepteur du système RFID dont la transmission de l'identificateur aux récepteurs avec antenne (6) se passe sans fil.

4. Dispositif selon la revendication 1, **caractérisé en ce que** lesdits récepteurs avec antenne (6) localisés dans le plateau (1) sont répartis sous forme de matrice et éloignés l'un de l'autre d'au moins 2 cm.

5. Dispositif selon la revendication 1, **caractérisé en ce que** dans chacun des coins du plateau (1), sur ladite rallonge se trouvent les caméras (5) constituant le système d'analyse des mouvements des membres supérieurs.

6. Dispositif selon la revendication 1, **caractérisé en ce que** lesdites rallonges sur lesquelles sont placées les caméras (5) sont de 5 cm à 50 cm de longueur.

7. Dispositif selon la revendication 1, **caractérisé en ce que** la largeur et la longueur des côtés du plateau (1) sont comprises entre 50 cm et 150 cm.

8. Dispositif selon la revendication 1, **caractérisé en ce que** ledit élément du casse-tête (7) est d'au moins 2 cm de hauteur.

9. Dispositif selon la revendication 1, **caractérisé en ce que** la communication entre lesdits éléments du casse-tête (7) et ledit contrôleur (2) se passe sans fil.

10. Dispositif selon la revendication 1, **caractérisé en ce que** la communication entre lesdites caméras (5) et ledit contrôleur (2) se passe par fil.

11. Dispositif selon la revendication 1, **caractérisé en ce que** la communication entre ladite caméra (9) et ledit contrôleur (2) se passe par fil.
